# EUROPÄISCHE PATENTSCHRIFT

(11) **EP 2 070 490 B1**
(45) Veröffentlichungstag und Bekanntmachung des Hinweises auf die Patenterteilung: **13.02.2019**
(21) Anmeldenummer: 08021409.1
(22) Anmeldetag: 10.12.2008
(51) Int. Cl.: A61F 2/24, A61B 17/12

(54) **Ummantelung zur Wiederherstellung der Klappenfunktion variköser Venen**
Sheath for restoring the valve functionality of varicose veins
Gaine destinée à la reproduction de la fonction de clapets de veines variqueuses

(30) Priorität: 10.12.2007 DE 102007061301
(43) Veröffentlichungstag der Anmeldung: 17.06.2009
(60) Teilanmeldung: 11193306.5
(73) Patentinhaber: AESCULAP AG, 78532 Tuttlingen (DE)
(72) Erfinder: Goldmann, Helmut, 78532 Tuttlingen/Donau (DE)
(74) Vertreter: Patentanwälte Ruff, Wilhelm, Beier, Dauster & Partner mbB

(56) Entgegenhaltungen:
- WO-A-95/05122
- WO-A-2004/026178
- WO-A1-03/094796
- DE-A1- 19 912 648
- US-A- 5 147 389

## Beschreibung

Die vorliegende Erfindung betrifft eine Ummantelung zur Wiederherstellung der Klappenfunktion variköser Venen und ihre Verwendung in der Chirurgie.

Die chronisch venöse Insuffizienz der unteren Extremitäten ist eine in der Bevölkerung weit verbreitete Erkrankung. Ursache hierfür ist häufig eine gestörte Venenklappenfunktion. Neuere Untersuchungen belegen, dass die Pathogenese für variköse Venen ursächlich mit veränderten elastischen Eigenschaften innerhalb der Gefäßwand zusammenhängt, die zu einer Dilatation der Venenwand führen. Infolge der Dilatation können die Venenklappen nicht mehr schließen, wodurch es zu einem vermehrten Blutrückfluss in die betreffenden Venen und schließlich zur Entstehung einer Varikose kommt. Vor allem das Nicht-Schließen der Mündungsklappe der Vena saphena magna (VSM) ist häufig Ausgangspunkt einer primären Varikose.

Das Spektrum an Therapiemöglichkeiten für die Stamminsuffizienz der Vena saphena magna reicht von konservativen Maßnahmen, wie etwa Kompressionsstrümpfen, Physio- und Pharmakotherapie, über minimalinvasive Verfahren bis hin zu operativen Verfahren. Die klassische operative Behandlung besteht in der Entfernung (Stripping) der insuffizienten Venen. In jüngster Zeit gelangen jedoch zunehmend auch minimalinvasive Verfahren, wie beispielsweise die endovaskuläre Lasertherapie (ELT) und die Radiofrequenzobliteration (RFO), zum Einsatz. Ein Nachteil der operativen bzw. minimal-invasiven Verfahren besteht allerdings darin, dass die betreffenden Venen aus dem Körper entfernt bzw. im Körper verödet werden.

Der allgemeine Wunsch nach Erhaltung von venösem Bypassmaterial für die koronare Bypasschirurgie sowie das Bestreben nach Minimierung des Operationstraumas haben zur Entwicklung von verschiedenen Venen erhaltenden Operationsverfahren geführt. In diesem Zusammenhang ist vor allem die sogenannte extraluminale Valvuloplastie der VSM-Mündungsklappe zu nennen. Die Klappenfunktion wird bei dieser Behandlungsmethode dadurch wieder hergestellt, dass eine Manschette um die dilatierte Klappenregion gelegt wird. Durch diese Operationstechnik können pathologische Refluxe in die Venen der unteren Extremitäten beseitigt werden, ohne die Venen selbst zu entfernen. Beispiele für derartige Manschetten sind aus der WO 88/00454 A1, US 5,147,389, US 4,904,254 und der WO 02/076305 A1 bekannt. Nachteilig bei den dort beschriebenen Manschetten ist allerdings neben ihrer aufwendigen und damit teuren Herstellungsweise vor allem die Tatsache, dass die dort beschriebenen Manschetten aus wenig oder gar nicht dehnbaren Materialien bestehen, wodurch eine venenadäquate Dehnbarkeit der Manschette nicht gegeben ist.

Aus der WO 95/05122 A1 ist ein extraluminales Medizinprodukt zur Korrektur insuffizienter Venenklappen bekannt. Ein extraluminaler, mehrteilig aufgebauter Stent, welcher vor allem für eine Verwendung im Zusammenhang mit der Aorta vorgesehen ist, ist aus der WO 2004/026178 A2 bekannt. Die DE 199 12 648 A1 offenbart ein sprühvliesförmig ausgestaltetes Implantat zur Verwendung in der Chirurgie. Ein tubuläres Implantat mit Kompositstruktur zur Behandlung von Blutgefäßklappen ist aus der WO 03/094796 A1 bekannt.

Eine vergleichsweise einfache Venenmanschette aus Dacron® (Polyethylenterephthalat) wurde von Mumme et al. (Phlebologie 2004; 33: 149 bis 155) erfolgreich in klinischen Studien für die extraluminale Venenklappenrekonstruktion eingesetzt. Problematisch ist jedoch auch bei diesem Manschettenmaterial, dass es keine elastische Dehnbarkeit aufweist. Zudem ist allgemein bekannt, dass Dacron® Fremdkörperreaktionen, insbesondere fibrotische Reaktionen, auslösen kann (S. Raju, ANN. SURG. (1983) 197, 688 bis 697).

Die Erfindung stellt sich daher die Aufgabe, eine Ummantelung für variköse Venen zur Verfügung zu stellen, welche aus dem Stand der Technik bekannte Probleme überwindet. Die Ummantelung soll insbesondere eine venenadäquate Elastizität aufweisen, bioverträglich sowie einfach und kostengünstig herstellbar sein.

Diese Aufgabe wird gelöst durch eine Ummantelung mit einer Längs- und einer Querrichtung zur Wiederherstellung der Klappenfunktion variköser Venen in Form eines flächigen, flexiblen Materialstücks, wobei das Materialstück ein non-woven Material aufweist.

Durch die Erfindung wird eine Ummantelung oder ein Venen-Patch bzw. eine Venenmanschette zur Venenklappenrekonstruktion variköser Venen in der Human- und/oder Veterinärmedizin bereitgestellt. Die Ummantelung eignet sich insbesondere zur Mündungsklappenrekonstruktion einer varikösen Vena saphena magna. Wie bereits einleitend erwähnt, ist eine stamminsuffiziente Vena saphena magna in den meisten Fällen ätiologischer Ausgangspunkt für die Entstehung einer primären Varikose. Zur Wiederherstellung eines intakten Venenklappenverschlusses wird die erfindungsgemäße Ummantelung in vorteilhafter Weise manschettenförmig um die variköse Vene gelegt. Die Manschette wirkt hierbei als eine Art innerer Kompressionstrumpf der den maßgeblichen Venenabschnitt vorzugsweise vollständig ummantelt. Unter ständiger Kontrolle des Blutrückflusses, meistens über einen nicht ligierten venösen Seitenast, kann die Manschette und damit der Lumendurchmesser der Vene stetig enger gestellt werden, bis ein Blutrückfluss in die Vene nicht mehr nachweisbar ist. Anschließend wird die Ummantelung in der Regel an ihren sich berührenden oder gegebenenfalls überlappenden Rändern fixiert. Die Ränder können beispielsweise verklebt und/oder vernäht werden.

Das Materialstück besitzt eine größere Länge als Breite. Das Materialstück selbst kann insbesondere als Streifen vorliegen. Das Materialstück weist Fixierelemente zur Fixierung der Ummantelung auf einer Vene auf. Bei den Fixierelementen handelt es sich um schenkelförmige Fortsätze des Materialstücks, wobei das Materialstück vorzugsweise zwei schenkelförmige Fortsätze aufweist. Die Fixierelemente können insbesondere für eine Fixierung der Ummantelung im Bereich einer natürlichen Veneneinmündungsregion, beispielsweise der sogenannten Krosseregion, vorgesehen sein. Bei der Krosseregion handelt es sich um die natürliche Veneneinmündung der Beinvene Vena saphena magna in die tiefer liegende Vena femoralis (auch als Femoralvene bezeichnet). Zur Wiederherstellung der Klappenfunktion einer varikösen Vena saphena magna ist die Ummantelung gewöhnlich mit der Femoralvene fixiert. Als Fixierungsmethoden kommen alle dem Fachmann geläufigen Fixierungstechniken, beispielsweise Kleben und/oder Nähen, in Betracht.

Das Materialstück besitzt eine in etwa rechteckige Form und weist an einer Schmalseite einen speziellen Zuschnitt auf. Dieser Zuschnitt ist mit besonderem Vorteil derart ausgebildet, dass die erfindungsgemäße Ummantelung im Bereich einer natürlichen Veneneinmündungsregion platziert werden kann. Bei dem speziellen Zuschnitt handelt es sich um einen U-förmigen Ausschnitt, dessen Breite dem Durchmesser der zu umfassenden Vene angepasst ist. Bevorzugt besitzt der U-förmige Ausschnitt eine Breite zwischen 5,8 und 8,2 mm, insbesondere eine Breite von ca. 6 mm.

In einer weitergehenden Ausführungsform ist das Materialstück in seinen Dimensionen der varikösen Vene, deren Klappenfunktion wiederhergestellt werden soll, angepasst. Das Materialstück kann eine Länge zwischen 3 und 5 cm, vorzugsweise von ca. 4 cm, und eine Breite zwischen 1 und 3 cm, bevorzugt von ca. 2 cm, aufweisen.

Das Materialstück ist weiterhin vorzugsweise dehnbar, insbesondere im Wesentlichen elastisch, ausgebildet. Mit besonderem Vorteil weist das Materialstück ein Dehnungsverhalten auf, das im Wesentlichen der Dehnbarkeit von gesunden Venen, insbesondere einer intakten Vena saphena magna, entspricht. Die Dehnbarkeit von gesunden bzw. intakten Venen wird auch als Compliance bezeichnet.

Besonders bevorzugt ist das Materialstück, insbesondere das non-woven Material, in Querrichtung vorgedehnt. Durch die Vordehnung erhält das Materialstück mit besonderem Vorteil ein Druck-Dehnungsverhalten, welches vorzugsweise in etwa der Dehnbarkeit von intakten Venen entspricht. Wird das non-woven Material der erfindungsgemäßen Ummantelung in Querrichtung vorgedehnt, kann dies in vorteilhafter Weise zu einer Verstärkung und damit Stabilisierung der Ummantelung führen. Der Grund hierfür ist insbesondere die Neuausrichtung der Fasern des non-woven Materials. Diese verlieren durch die Vordehnung, einer kalten Verstreckung vergleichbar, zumindest zum Teil ihre ursprüngliche Orientierung untereinander und richten sich neu aus. Durch die Faserneuausrichtung kann es zu einer Verstärkung des non-woven Materials und damit insgesamt der Ummantelung kommen. Normalerweise ist das Materialstück kalt vorgedehnt.

Vorzugsweise ist das Materialstück, insbesondere das non-woven Material, mechanisch vorgedehnt. In einer weitergehenden Ausführungsform ist das Materialstück in Querrichtung durch eine Zugkraft zwischen 1 und 20 N/cm, insbesondere 4 und 18 N/cm, vorzugsweise 8 und 16 N/cm, vorgedehnt.

Das non-woven Material liegt in Form eines Sprühvlieses vor. Erfindungsgemäß kann es vorgesehen sein, dass das Sprühvlies Fasern aufweist, die miteinander verklebt sind. Zur Herstellung des Vlieses wird normalerweise eine Lösung des non-woven Materials in einem leicht flüchtigen Lösungsmittel verwendet. Als Lösungsmittel kommen daher insbesondere halogenierte Lösungsmittel in Betracht. Die Lösungsmittel können dabei teil- und/oder perhalogeniert sein. Besonders bevorzugt sind chlorierte und/oder fluorierte Lösungsmittel, beispielsweise Chloroform und/oder Dichlormethan. Zur Herstellung einer Sprühvliesstruktur wird die Lösung des non-woven Materials in der Regel auf eine ebene Auftragsfläche aufgesprüht. Das Aufsprühen selbst erfolgt normalerweise mit Hilfe einer geeigneten Sprüh- oder Zerstäubervorrichtung, beispielsweise einer Sprühpistole oder -düse. Der Sprühweg ist dabei so bemessen, dass eine Faserbildung des non-woven Materials aus der Lösung beim Durchlaufen des Sprühweges stattfindet. Der Sprühweg kann beispielsweise zwischen 10 und 75 cm, insbesondere 15 und 20 cm, betragen. Das Lösungsmittel verflüchtigt sich dagegen in der Regel beim Durchlaufen des Sprühweges, so dass sich auf der Auftragsfläche non-woven Materialfasern abscheiden, welche sich aufgrund einer gewissen Restfeuchte vorzugsweise unter Bildung einer dreidimensionalen Faserstruktur (Vliesstruktur) miteinander verkleben. Ein besonderer Vorteil der Sprühvliestechnik besteht darin, dass sich der mittlere Porendurchmesser im Sprühvlies in Abhängigkeit vom Sprühweg einstellen lässt. Je länger der Sprühweg ist, desto größer ist der mittlere Porendurchmesser im fertigen Sprühvlies. Auf diese Weise können Vliesstrukturen mit Porengradienten hergestellt werden.

Das Materialstück ist bei einer weiteren Ausführungsform mehrschichtig, insbesondere zwei- oder dreischichtig, aufgebaut.

Weiterhin kann das non-woven Material Fasern mit einer Faserstärke zwischen 0,01 und 20 µm, insbesondere 0,1 und 10 µm, vorzugsweise 0,5 und 5 µm, aufweisen.

In einer weitergehenden Ausführungsform ist das non-woven Material ein unter physiologischen Bedingungen im Wesentlichen nicht resorbierbares Polymer. Das non-woven Material ist bevorzugt ein bioverträgliches Polymer. Vorzugsweise handelt es sich bei dem non-woven Material um ein Polyurethan. Polyurethan besitzt den Vorteil, dass es ein außerordentlich bioverträgliches Material darstellt, welches zudem eine elastische Dehnbarkeit besitzt, die im Wesentlichen der natürlichen Dehnbarkeit einer gesunden Venenwand entspricht. Erfindungsgemäß ist es insbesondere vorgesehen sein, dass das non-woven Material ein thermoplastisches Polyurethan ist. Zweckmäßigerweise ist das Polyurethan in organischen Lösungsmitteln, insbesondere in flüchtigen organischen Lösungsmitteln, löslich. Insbesondere handelt es sich bei dem non-woven Material um ein lineares Polyurethan, vorzugsweise lineares aliphatisches Polyurethan. Bei den in Frage kommenden Polyurethanen kann es sich vor allem um Polyurethane handeln, welche aus makro- und/oder niedermolekularen aliphatischen Diolen sowie aliphatischen Diisocyanaten hergestellt sind. Erfindungsgemäß ist es insbesondere vorgesehen, dass es sich bei den makromolekularen Diolen um Polycarbonate, insbesondere um 1,6-Hexandiolpolycarbonat, handelt. Als niedermolekulare Diole kommen bevorzugt 2,2,4-Trimethylhexandiol, 2,4,4-Trimethylhexandiol und/oder 1,4-Butandiol in Betracht. Als aliphatische Diisocyanate werden bevorzugt cycloaliphatische Diisocyanate, insbesondere 4,4'-Dicyclohexylmethan-diisocyanat und/oder 1,4-Cyclohexyl-diisocyanat, verwendet. Bezüglich weiterer in Frage kommende Polyurethane wird auf die DE 36 43 465 A1, DE 33 18 730 A1 und die DE 41 07 284 A1 verwiesen, deren Offenbarungsgehalt jeweils durch Bezugnahme zum Inhalt der vorliegenden Beschreibung gemacht wird.

Das non-woven Material kann weiterhin ein Polyurethan mit einem Molekulargewicht zwischen 5.000 und 50.000 Dalton, insbesondere 20.000 und 40.000 Dalton, sein.

In einer besonders vorteilhaften Ausführungsform ist das Materialstück dünner ausgebildet als die aus dem Stand der Technik bekannten Manschetten. Vorzugsweise weist das Materialstück eine Dicke auf, die eine problemlose Fixierung der Ummantelung, insbesondere durch Vernähen, auf einer natürlichen Vene erlaubt. Bevorzugt weist das Materialstück eine Dicke zwischen 0,1 und 0,5 mm, insbesondere 0,15 und 0,3 mm, auf. Ein dünnes und insbesondere elastisch dehnbar ausgebildetes Materialstück verbessert zudem mit besonderem Vorteil die allgemeine Handhabung durch den Chirurgen, wodurch Operationszeiten und - risiken verringert werden können.

Das Materialstück ist bei einer weiteren Ausführungsform porös. Das Materialstück kann insbesondere eine durchgängige Porosität aufweisen. Bevorzugt weist das Materialstück Poren mit einem Durchmesser zwischen 0,1 und 100 µm, insbesondere 0,5 und 50 µm, vorzugsweise 1 und 10 µm, auf.

Erfindungsgemäß kann die Ummantelung zumindest eine Verstärkung aufweisen. Bei der Verstärkung kann es sich beispielsweise um ein textiles Netz, ein Metalldraht oder eine Folie handeln. Die Verstärkung ist vorzugsweise der Compliance von natürlichen gesunden Venen angepasst.

Erfindungsgemäß kann es zu dem vorgesehen sein, dass die Ummantelung Wirkstoffe, insbesondere antimikrobielle Wirkstoffe, aufweist. Die Wirkstoffe selbst können dabei in Form von Partikeln vorliegen, deren Durchmesser bevorzugt zwischen 50 und 100 nm liegt. Die Wirkstoffpartikel können auch zu Agglomeraten verbunden sein. Die Agglomeratgröße kann insbesondere zwischen 5 und 10 µm liegen. Als Wirkstoffe kommen vor allem Metalle oder Metalllegierungen mit antimikrobieller Wirkung in Frage, wobei metallisches Silber besonders bevorzugt ist. Weiterhin können die Wirkstoffe gegebenenfalls zusammen mit anderen Materialien in Form einer Schicht auf der Ummantelung aufgebracht sein. Beispielsweise können die Wirkstoffe zusammen mit resorbierbaren Materialien, insbesondere Gelatine und/oder Kollagen, in Form einer resorbierbaren Schicht auf der Oberfläche der Ummantelung vorliegen. Besonders bevorzugt weist die Ummantelung eine Silberschicht auf. Die Dicke der Silberschicht kann grundsätzlich im Bereich zwischen 1.000 und 4.000 Å, insbesondere 1.000 und 2.500 Å, liegen.

Gemäß einer weiteren Ausführungsform liegt die Ummantelung in verpackter Form vor. Die Ummantelung ist mit besonderem Vorteil sterilisierbar und liegt vorzugsweise in sterilisierter Form vor.

In einer bevorzugten Ausführungsform besteht das Materialstück aus dem non-woven Material. Bezüglich weiterer Einzelheiten und Merkmale des non-woven Materials wird auf die bisherige Beschreibung Bezug genommen.

Die Erfindung betrifft weiterhin ein Kit, insbesondere zur Verwendung in der extraluminalen Valvuloplastie, welcher zumindest die Ummantelung umfasst. Als weitere Komponente umfasst das Kit vorzugsweise eine bioverträgliche Klebstoffzusammensetzung. Die Klebstoffzusammensetzung selbst kann beispielsweise aus n-Alkyl-Cyanacrylate bestehen. Bevorzugt besteht die Klebstoffzusammensetzung aus n-Butyl-2-Cyanacrylat (Histoacryl®). Als Alternative zu einer Klebstoffzusammensetzung oder in Kombination dazu kann das Kit ein Nahtmaterial, beispielsweise einen Polypropylenfaden, aufweisen.

Die vorliegende Erfindung betrifft schließlich auch die Verwendung der Ummantelung zur Bereit- oder Herstellung eines chirurgischen Implantats zur Wiederherstellung der Venenklappenfunktion variköser Venen, insbesondere einer varikösen Vena saphena magna. Die erfindungsgemäße Ummantelung eignet sich dabei insbesondere für einen Einsatz in der extraluminalen Valvuloplastie.

Weitere Einzelheiten und Merkmale der vorliegenden Erfindung ergeben sich aus der nachfolgenden Beschreibung bevorzugter Ausführungsformen anhand der Figuren in Kombination mit den Unteransprüchen. In diesen Ausführungsformen können einzelne Merkmale der Erfindung allein oder in Kombination mit anderen Merkmalen verwirklicht sein. Sämtliche Figuren werden hiermit durch ausdrückliche Bezugnahme zum Inhalt dieser Beschreibung gemacht.

In den Figuren zeigen:
- Figur 1:: eine Ausführungsform einer erfindungsgemäßen Ummantelung,
- Figur 2:: die Krosseregion,
- Figur 3:: die Mündungsklappenregion der Vena saphena magna nach einer durchgeführten extraluminalen Valvuloplastie.

### Figurenbeschreibung

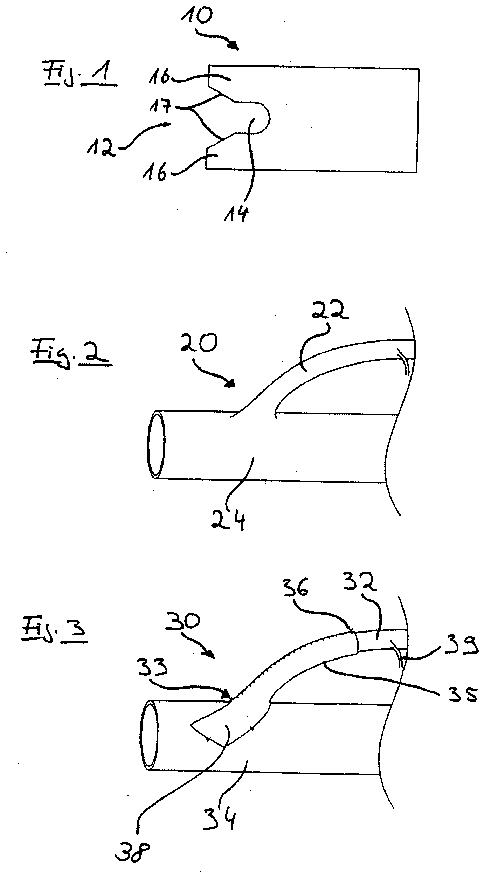

Die Figur 1 zeigt schematisch eine erfindungsgemäße Ummantelung **10,** welche vollständig als poröser Sprühvlies ausgebildet ist. Bei dem Sprühvliesmaterial handelt es sich um Polyestercarbonaturethan. Die Ummantelung **10** ist ein etwa 4 x 2 cm großes, rechteckiges Materialstück, dessen eine Schmalseite **12** einen U-förmigen Ausschnitts **14** aufweist, wodurch die Ummantelung **10** zwei schenkelförmige Fortsätze **16** erhält. Die Breite des Ausschnitts **14** beträgt ca. 6 mm. Die Schmalseite **12** weist zudem in Richtung des U-förmigen Ausschnitts **14** abgeschrägte Verläufe **17** auf. Durch diesen speziellen Zuschnitt kann die Ummantelung **10** beispielsweise genau in die Mündungsklappenregion einer insuffizienten Vena saphena magna (VSM), insbesondere in den Winkel zwischen der VSM-Einmündung und der tiefer liegenden Vena femoralis, platziert werden (vgl. hierzu auch die in Figur 2 dargestellte Krosseregion). Die Dicke der Ummantelung **10** beträgt etwa 0,15 mm. Dadurch lässt sich die Ummantelung **10** besonders einfach im Bereich einer natürlichen Veneneinmündungsregion, insbesondere mit der Vena femoralis, vernähen.

Figur 2 zeigt schematisch die natürliche Einmündungsregion **20** der Vena saphena magna (VSM) **22** in die tiefer liegende Beinvene Vena femoralis **24.** Die Einmündungsregion **20** wird auch als Krosseregion bezeichnet. Die Venenklappe der Vena saphena magna **22** befindet sich in etwa dort, wo die VSM **22** direkt in die tiefer liegende Femoralvene **24** einmündet. Ist die Klappenfunktion der Vena saphena magna **22** beeinträchtigt, gewöhnlich weil die Venensegel der Klappe nicht mehr schließen, kommt es zu einem vermehrten Blutrückfluss in die Vena saphena magna **22.** Dadurch kommt es zu einem vermehrten Blutstau in den unteren Extremitäten, wodurch der Blutdruck und damit allgemein das Risiko für eine Varikose steigt.

Figur 3 zeigt schematisch die Krosseregion **30** nach einer durchgeführten extraluminalen Valvuloplastie, wie sie in ähnlicher Weise für eine Ummantelung aus Polyester bekannt ist. Die Vena saphena magna **32** ist ausgehend von ihrer Einmündung **33** in die darunter liegende Vena femoralis **34** über einen bestimmten Längsabschnitt im Wesentlichen vollständig mit der erfindungsgemäßen Ummantelung **35** aus einem Polyurethan-Vlies umwickelt. Die Ummantelung **35** ist an ihren sich berührenden oder gegebenenfalls überlappenden Rändern mit einem Polypropylenfaden **36** vernäht. Des Weiteren ist die Ummantelung **35** über zwei schenkelförmige Fortsätze **38** mit der Vena femoralis **34** vernäht. Der nicht ligierte Seitenast **39** dient als Indikatorvene zur Kontrolle des Blutrückflusses während des Engerstellens des Lumendurchmessers der Vena saphena magna mittels der Ummantelung **35.**

## Patentansprüche

1. Ummantelung (10; 35) mit einer Längsrichtung und einer Querrichtung zur Wiederherstellung der Venenklappenfunktion variköser Venen in Form eines flächigen, flexiblen Materialstücks, wobei das Materialstück ein non-woven Material aufweist, **dadurch gekennzeichnet, dass** das Materialstück schenkelförmige Fortsätze (16; 38) aufweist, das Materialstück eine in etwa rechteckige Form besitzt und an einer Schmalseite (12) einen U-förmigen Ausschnitt (14) aufweist, dessen Breite dem Durchmesser der zu umfassenden Vene angepasst ist, und das non-woven Material in Form eines Sprühvlieses vorliegt.

2. Ummantelung (10; 35) nach Anspruch 1, **dadurch gekennzeichnet, dass** das Materialstück zwei schenkelförmige Fortsätze (16; 38) aufweist.

3. Ummantelung (10; 35) nach Anspruch 1 oder 2, **dadurch gekennzeichnet, dass** das Materialstück dehnbar, insbesondere im Wesentlichen elastisch, ausgebildet ist.

4. Ummantelung (10; 35) nach einem der vorhergehenden Ansprüche, **dadurch gekennzeichnet, dass** das Materialstück ein Dehnungsverhalten aufweist, das im Wesentlichen der Dehnbarkeit von gesunden Venen entspricht.

5. Ummantelung (10; 35) nach einem der vorhergehenden Ansprüche, **dadurch gekennzeichnet, dass** das Materialstück in Querrichtung vorgedehnt ist.

6. Ummantelung (10; 35) nach einem der vorhergehenden Ansprüche, **dadurch gekennzeichnet, dass** das Materialstück in Querrichtung durch eine Zugkraft zwischen 1 und 20 N/cm, insbesondere 4 und 18 N/cm, vorzugsweise 8 und 16 N/cm, vorgedehnt ist.

7. Ummantelung (10; 35) nach einem der vorhergehenden Ansprüche, **dadurch gekennzeichnet, dass** das non-woven Material ein unter physiologischen Bedingungen im Wesentlichen nicht resorbierbares Polymer ist.

8. Ummantelung (10; 35) nach einem der vorhergehenden Ansprüche, **dadurch gekennzeichnet, dass** es sich bei dem non-woven Material um ein Polyurethan, insbesondere lineares Polyurethan, vorzugsweise lineares aliphatisches Polyurethan, handelt.

9. Ummantelung (10; 35) nach einem der vorhergehenden Ansprüche, **dadurch gekennzeichnet, dass** die Ummantelung (10; 35) eine Verstärkung aufweist.

10. Ummantelung (10; 35) nach einem der vorhergehenden Ansprüche zur Mündungsklappenrekonstruktion einer varikösen Vena saphena magna.

11. Kit, insbesondere zur Verwendung in der extraluminalen Valvuloplastie, umfassend zumindest eine Ummantelung (10; 35) nach einem der vorhergehenden Ansprüche und vorzugsweise eine bioverträgliche Klebstoffzusammensetzung für die Ummantelung (10; 35).

## Claims

1. Sheathing (10; 35) having a longitudinal direction and a transverse direction for restoring the function of venous valves of varicose veins in the form of a planar, flexible piece of material, wherein the piece of material includes a nonwoven material,
**characterized in that**
the piece of material has shank-like projections (16; 38), the piece of material has an approximately rectangular shape and has on one narrow side (12) a U-shaped cutout (14) whose width is adapted to the diameter of the vein to be enveloped, and the nonwoven material is in the form of a sprayed web.

2. Sheathing (10; 35) according to claim 1, **characterized in that** the piece of material has two shank-like projections (16; 38).

3. Sheathing (10; 35) according to claim 1 or 2, **characterized in that** the piece of material is designed to be extensible, in particular substantially elastic.

4. Sheathing (10; 35) according to any of the preceding claims, **characterized in that** the piece of material has an extensibility which substantially corresponds to the extensibility of healthy veins.

5. Sheathing (10; 35) according to any of the preceding claims, **characterized in that** the piece of material is prestretched in the transverse direction.

6. Sheathing (10; 35) according to any of the preceding claims, **characterized in that** the piece of material is prestretched in the transverse direction by a tensile force of between 1 and 20 N/cm, in particular 4 and 18 N/cm, preferably 8 and 16 N/cm.

7. Sheathing (10; 35) according to any of the preceding claims, **characterized in that** the nonwoven material is a polymer which is substantially non-absorbable under physiological conditions.

8. Sheathing (10; 35) according to any of the preceding claims, **characterized in that** the nonwoven material is a polyurethane, in particular linear polyurethane, preferably linear aliphatic polyurethane.

9. Sheathing (10; 35) according to any of the preceding claims, **characterized in that** the sheathing (10; 35) has a reinforcement.

10. Sheathing (10; 35) according to any of the preceding claims for terminal valve reconstruction of a varicose great saphenous vein.

11. Kit, in particular for use in extraluminal valvuloplasty, comprising at least one sheathing (10; 35) according to any of the preceding claims and preferably a biocompatible adhesive composition for the sheathing (10; 35).

## Revendications

1. Gaine (10; 35) présentant une direction longitudinale et une direction transversale pour la reproduction de la fonction de clapet de veine de veines variqueuses sous la forme d'une pièce de matière plate, flexible, dans laquelle la pièce de matière présente une matière non tissée, **caractérisée en ce que** la pièce de matière présente des prolongements en forme de branches (16; 38), la pièce de matière possède une forme sensiblement rectangulaire et présente sur un côté étroit (12) une découpe en forme de U (14), dont la largeur est adaptée au diamètre de la veine à gainer, et la matière non tissée se présente sous la forme d'un non tissé du type lié par pulvérisation.

2. Gaine (10; 35) selon la revendication 1, **caractérisée en ce que** la pièce de matière présente deux prolongements en forme de branches (16; 38).

3. Gaine (10; 35) selon la revendication 1 ou 2, **caractérisée en ce que** la pièce de matière est extensible, notamment est essentiellement élastique.

4. Gaine (10; 35) selon l'une quelconque des revendications précédentes, **caractérisée en ce que** la pièce de matière présente un comportement extensible, qui correspond essentiellement à l'extensibilité de veines saines.

5. Gaine (10; 35) selon l'une quelconque des revendications précédentes, **caractérisée en ce que** la pièce de matière est pré-étirée en direction transversale.

6. Gaine (10; 35) selon l'une quelconque des revendications précédentes, **caractérisée en ce que** la pièce de matière est pré-étirée en direction transversale par une force de traction comprise entre 1 et 20 N/cm, en particulier 4 et 18 N/cm, de préférence entre 8 et 16 N/cm.

7. Gaine (10; 35) selon l'une quelconque des revendications précédentes, **caractérisée en ce que** la matière non tissée est un polymère essentiellement non résorbable dans des conditions physiologiques.

8. Gaine (10; 35) selon l'une quelconque des revendications précédentes, **caractérisée en ce que** la matière non tissée est un polyuréthane, en particulier un polyuréthane linéaire, de préférence un polyuréthane linéaire aliphatique.

9. Gaine (10; 35) selon l'une quelconque des revendications précédentes, **caractérisée en ce que** la gaine (10; 35) présente un renfort.

10. Gaine (10; 35) selon l'une quelconque des revendications précédentes, pour la reconstruction de la valvule ostiale d'une veine grande saphène variqueuse.

11. Kit, en particulier utilisable en valvuloplastie extraluminale, comprenant au moins une gaine (10; 35) selon l'une quelconque des revendications précédentes et de préférence une composition adhésive biocompatible pour la gaine (10; 35).
